## Europäisches Patentamt

## European Patent Office

## Office européen des brevets

(19)

(11) Veröffentlichungsnummer: **0 010 245**
**B1**

(12) **EUROPÄISCHE PATENTSCHRIFT**

(45) Veröffentlichungstag der Patentschrift:
05.05.82

(21) Anmeldenummer: 79103841.7

(22) Anmeldetag: 08.10.79

(51) Int. Cl.³: **C 07 D 201/04, B 01 J 21/02, B 01 J 21/20**

(54) Verfahren zur Herstellung von epsilon-Caprolactam durch katalytische Umlagerung von Cyclohexanonoxim.

(30) Priorität: 14.10.78 DE 2844880

(43) Veröffentlichungstag der Anmeldung:
30.04.80 Patentblatt 80/9

(45) Bekanntmachung des Hinweises auf die Patenterteilung:
05.05.82 Patentblatt 82/18

(84) Benannte Vertragsstaaten:
BE CH DE FR GB IT NL

(56) Entgegenhaltungen:
DE-A-1 953 153
DE-A1-2 641 381
DE-A1-2 641 389
DE-A1-2 641 408
DE-A1-2 641 429
DE-B-1 670 902
DE-B2-1 951 158
DE-B2-2 003 460
NL-A-6 811 222

Die Akte enthält technische Angaben, die nach dem
Eingang der Anmeldung eingereicht wurden und die nicht
in dieser Patentschrift enthalten sind.

(73) Patentinhaber: BASF Aktiengesellschaft,
Carl-Bosch-Strasse 38, D-6700 Ludwigshafen (DE)

(72) Erfinder: Fuchs, Hugo, Dr., Egellstrasse 28,
D-6700 Ludwigshafen (DE)
Erfinder: Brand, Uwe, Rheingoldstrasse 12,
D-6841 Rosengarten (DE)
Erfinder: Horn, Peter, Dr., Im Bruennel 20,
D-6945 Hirschberg (DE)

ACTORUM AG.

Verfahren zur Herstellung von ε-Caprolactam durch katalytische Umlagerung von Cyclohexanonoxim

Gegenstand der Erfindung ist ein Verfahren zur Herstellung von ε-Caprolactam durch katalytische Umlagerung von Cyclohexanonoxim in der Gasphase bei einer Temperatur von 230 bis 450°C an Bortrioxid enthaltenden Trägerkatalysatoren in der Wirbelschicht.

Bei der katalytischen Umlagerung von Cyclohexanonoxim lagern sich kohlenstoffhaltige Zersetzungsprodukte auf der Kontaktmasse ab, wodurch deren Wirkungsdauer begrenzt wird. Aus der DE-A-1 670 902 ist bekannt, dass man unwirksam gewordene Katalysatoren durch Erhitzen auf 500 bis 900°C in Gegenwart von Luft durch Abbrennen der Abscheidungen regeneriert. Wie dort ausgeführt wird, ist es jedoch notwendig, etwaige Verluste an Bortrioxid vor der Wiederverwendung des Katalysators zu ergänzen. Deshalb wird der aufgewirbelten Katalysatormasse bei einer Temperatur von 300 bis 600°C Borsäureester zugeführt, wobei sich dieser zersetzt und Bortrioxid auf der Katalysatormasse ablagert. Hierzu bedarf es jedoch einer genauen Steuerung des Zersetzungsvorgangs, um die flüchtigen Borsäureester in dem Masse zu zersetzen wie erforderlich. Andererseits wird in der DE-A-1 670 902 bzw. in der äquivalenten NL-A-6 811 222 ausgeführt, dass man anstatt Borsäureester auch feinverteilte Borsäure oder feingemahlenes Bortrioxid in die aufgewirbelte Katalysatorschicht einleiten kann. Es wird darauf hingewiesen, dass es darauf ankommt die Bildung einer selbständigen Boroxidphase zu vermeiden die leicht zum Verbacken der Katalysatormasse führt.

Es war deshalb die technische Aufgabe gestellt, bei der katalytischen Umlagerung von Cyclohexanonoxim zum ε-Caprolactam die Wiederaufbereitung des Katalysators so zu gestalten, dass ein Verbacken der Katalysatormasse vermieden wird und zugleich eine gleichmässige Dosierung der katalytisch aktiven Verbindung erzielt wird, um eine gleichbleibende Aktivität des Katalysators zu erhalten.

Diese technische Aufgabe wird gelöst in einem Verfahren zur Herstellung von ε-Caprolactam durch katalytische Umlagerung von Cyclohexanonoxim in der Gasphase bei einer Temperatur von 230 bis 450°C an Bortrioxid enthaltenden Trägerkatalysatoren in der Wirbelschicht, wobei der Katalysator nach Massgabe seiner Erschöpfung der Wirbelschicht entnommen und mit molekularen Sauerstoff enthaltenden Gasen in aufgewirbeltem Zustand bei einer Temperatur von 600 bis 900°C behandelt und anschliessend vor Zurückführen in die Wirbelschicht dem Katalysator in aufgewirbeltem Zustand bei einer Temperatur von 300 bis 900°C Bortrioxid oder Borsäure zugesetzt wird, indem man Bortrioxid oder Borsäure mit einer Korngrösse von 0,05 bis 1,5 mm an mindestens 2 Stellen dem aufgewirbelten Katalysator zuführt.

Das neue Verfahren hat den Vorteil, dass ein Verbacken der Katalysatorschicht vermieden wird und eine gleichmässige Katalysatorqualität erreicht wird. Die erfindungsgemässe Arbeitsweise erlaubt einen störungsfreien Verfahrensablauf über lange Zeit. Ferner ist das neue Verfahren wenig aufwendig und gestattet eine zuverlässige Dosierung der katalytisch aktiven Verbindung.

Die Umlagerungsbedingungen für Cyclohexanonoxim zu ε-Caprolactam sind an sich bekannt. Das Cyclohexanonoxim wird dampfförmig oder flüssig oder in fester Form in die Wirbelschicht geleitet, wo sich der Katalysator auf Reaktionstemperatur befindet. Vorteilhaft hat das Cyclohexanonoxim einen Wassergehalt von 1 bis 10, insbesondere 3 bis 7 Gew.-%. Die Umlagerung wird bei Temperaturen von 230 bis 450°C, vorzugsweise 270 bis 370°C, durchgeführt. Man kann das Verfahren bei Normaldruck, bei vermindertem oder bei leichtem Überdruck ausführen. Sofern man verminderten Druck anwendet, wird der Druckbereich von 20 bis 200 Torr bevorzugt, führt man das Verfahren bei Überdruck durch, werden im allgemeinen Drücke über 2 bar nicht angewendet. Der Katalysator wird mit Inertgas, wie Kohlendioxid, Argon, Stickstoff, vorzugsweise Stickstoff, in wirbelnder Bewegung gehalten. Im allgemeinen wird das Inertgas in einer Menge von 5 bis 70 Volumenprozent, bezogen auf das Gasgemisch, bestehend aus Cyclohexanonoximdampf bzw. Caprolactam und Inertgas verwendet, je nachdem ob man das Verfahren bei Normal- oder Überdruck oder bei vermindertem Druck ausführt, wobei man bei höherem Druck auch mehr Inertgas verwendet.

Es ist auch zweckmässig, das Inertgas mit Temperaturen von 80 bis 300°C der Wirbelschicht zuzuführen und durch das in der Wirbelschicht erhitzte Inertgas die Umlagerungswärme abzuführen.

Als Katalysator verwendet man bekannte Katalysatoren bei denen Bortrioxid oder Borsäure, die unter Reaktionsbedingungen in Bortrioxid übergeht, auf Trägern aufgebracht sind. Als Träger sind insbesondere Aluminiumoxid in seinen verschiedenen Modifikationen, wie Tonerde, γ-Aluminiumoxid, Böhmit, ferner Kieselsäure oder Titandioxid oder Gemische derartiger Oxide oder von Verbindungen der Oxide untereinander, z.B. Aluminiumsilikate, geeignet. Das Gewichtsverhältnis von Bortrioxid zu Träger beträgt im allgemeinen von 1 : 9 bis 1 : 1. Bei bevorzugt verwendeten Katalysatoren beträgt der Anteil an Bortrioxid 25 bis 50 Gew.-%. Die Katalysatoren können ausserdem durch Zusätze, z.B. von Mangan, Kobalt oder Nickel in einer Menge bis zu 10 Gew.-%, bezogen auf $B_2O_3$ und berechnet als Metall modifiziert sein. Der Zusatz erfolgt bei der Herstellung in Form von Salzen, die beim Erhitzen die entsprechenden Oxide bilden, wie Nitrate oder fettsaure Salze. Dementsprechend liegen die Metalle im fertigen Katalysator als Oxide oder deren Verbindungen mit Bortrioxid vor. Die Katalysatoren werden in üblicher Weise hergestellt, z.B. werden die Träger mit Borsäure oder Ammoniumboratlösung getränkt, bei 50 bis 500°C getrocknet und anschliessend zur Umwandlung der aufgebrachten Verbindungen in die entsprechenden Mischphasen mit Boroxid bei 600 bis 1200°C geglüht. Die Katalysatoren werden in üblicher Weise geformt, z.B. durch Anteigen von Bortrioxid und Träger mit wenig Wasser, Vermengen im Kneter, Pressen der Masse zu Strängen oder Pillen, Trocknen und Glühen bei den angegebenen Temperaturen. Zweckmässig verwendet man Korngrössen von 0,05 bis 1,5 mm, insbesondere von 0,2 bis 1,0 mm. Die Höhe der Katalysatorschicht wird vorteilhaft so gewählt, dass die Verweilzeiten des Cyclohexanonoxims an der Katalysatorschicht von 0,01 bis 30 Sekuden, insbesondere von 0,1 bis 5 Sekunden, betragen.

Aus dem erhaltenen ε-caprolactamhaltigen Gas wird Caprolactam durch Abschrecken mit Caprolactam oder stufenweise zunächst durch Abschrecken mit ε-Caprolactam und dann mit Wasser in einer Kolonne abgeschieden. Das abgetrennte Inertgas wird nach Abscheiden von Wasser wieder der Wirbelschicht zugeführt.

Der Wirbelschicht wird periodisch oder vorteilhaft kontinuierlich Katalysator entnommen und bei 600 bis 900°C mit molekularen Sauerstoff enthaltendem Gas, insbesondere Luft, behandelt. Hierbei liegt der Katalysator in aufgewirbelter Form vor. Vorteilhaft verwendet man pro kg Katalysator im Regenerator 0,5 bis 10 kg molekularen Sauerstoff enthaltendes Gas. Die Behandlungszeit beträgt in der Regel von 10 Minuten bis 10 Stunden. In der Regel entnimmt man der Wirbelschicht stündlich den 0,1- bis 10fachen Teil des dort vorliegenden Katalysators je nach der zugeführten Menge an Cyclohexanonoxim.

Der so behandelte Katalysator wird nun, bevor er in die Wirbelschicht zurückgeleitet wird mit Bortrioxid oder Borsäure dotiert, um Verluste an Bortrioxid auszugleichen und die ursprüngliche katalytische Aktivität wieder herzustellen. Hierbei wird dem Katalysator bei einer Temperatur von 300 bis 900°C im aufgewirbelten Zustand Bortrioxid oder Borsäure in einer Korngrösse von 0,05 bis 1,5 mm an mindestens 2 Stellen gleichzeitig zugeführt. Vorteilhaft führt man Bortrioxid oder Borsäure an 2 bis 5 Stellen gleichzeitig zu.

Besonders vorteilhaft hat es sich erwiesen, wenn man Bortrioxid oder Borsäure gemischt mit Caprolactam oder Harnstoff zuführt. Solche Gemische enthalten vorzugsweise 10 bis 99 Gew.-% Bortrioxid oder Borsäure, berechnet als Bortrioxid. Die Korngrösse des zugeführten Gemisches beträgt vorteilhaft 0,1 bis 2 mm.

Bei der Dotierung des Katalysators mit den katalytisch aktiven Verbindungen wird dieser mit Luft in aufgewirbeltem Zustand gehalten, wobei die katalytisch aktiven Verbindungen zweckmässig mit einem Luftstrom in die auf- und abwirbelnde Katalysatormasse eingebracht werden. Die Menge der zugesetzten katalytisch aktiven Verbindungen richtet sich nach dem Verlust an Bortrioxid und beträgt im allgemeinen 0,1 bis 20 Gew.-%, bezogen auf die regenerierte Katalysatormasse.

Der so behandelte Katalysator wird dann vorzugsweise nach Abkühlen auf 400 bis 300°C wieder der Wirbelschicht zur Umlagerung von Cyclohexanonoxim zugeführt.

Caprolactam, das nach dem Verfahren der Erfindung hergestellt wird, eignet sich zur Herstellung von Polycaprolactam.

Das Verfahren nach der Erfindung sei in folgenden Beispielen veranschaulicht.

Beispiel 1

In einem Reaktor mit den Dimensionen 5 000 mm Länge und 1 200 mm Durchmesser werden 500 kg eines Katalysators bestehend aus 55% Aluminiumoxid und 45% Bortrioxid der Korngrösse 0,3 bis 1,0 mm vorgelegt. Stündlich werden 650 kg Stickstoff der durch eine elektrische Heizung auf 360°C gebracht wurde, über einen Lochboden in den Reaktor geblasen und dadurch der Katalysator zum Wirbeln gebracht. Über 2 Düsen werden nun 400 kg Cyclohexanonoxim pro Stunde mit einem Wassergehalt von 4 Gew.-% in die Wirbelschicht zudosiert. Die Eingangstemperatur des zum Wirbeln benötigten Stickstoffs wird in dem Masse gesenkt, wie die Temperatur im Reaktor ansteigt, so dass eine Reaktionstemperatur von 360°C gehalten wird. Die Reaktionsgase werden getrennt und das entstandene Caprolactam aufgearbeitet.

Dem Reaktor werden stündlich 200 kg Katalysator entnommen und einem Regenerator zugeleitet. Durch Einblasen von auf 700°C vorgeheizter Luft über einen Lochboden wird der Katalysator am Wirbeln gehalten. Die Innentemperatur beträgt ca. 800°C. Die benötigte Luftmenge beträgt ca. 700 kg/Std.

Die auf dem Katalysator befindlichen Verunreinigungen verbrennen. Der regenerierte Katalysator wird in einer weiteren Wirbelschicht durch Einblasen von kälterer Luft auf ca. 400°C abgekühlt. Die Dimensionen des Kühlreaktors sind 3 500 mm Länge und 1 400 mm Durchmesser. Im Abstand von 180 mm über dem Anströmboden werden über 2 Düsen stündlich 4 kg Borsäure der Korngrösse 0,3 bis 1 mm in die Wirbelschicht dosiert. Hierzu wird die Borsäure über eine Dosierschnecke einem Gasstrahlfeststofförderer zugeführt und mit ca. 50 kg Luft/Stunde in die Wirbelschicht gebracht.

Der Boroxidgehalt des Katalysators wird so bei 42 bis 44% gehalten. Ohne ständige Zufuhr von Borsäure verliert der Katalysator ca. 1% Boroxid pro Tag.

Verbackungen konnten über einen Zeitraum von 4 Wochen nicht festgestellt werden.

Beispiel 2

Man verfährt wie in Beispiel 1 beschrieben. Anstelle von Borsäure wird jedoch ein Gemisch aus Borsäure und Harnstoff im Gewichtsverhältnis 80 Gew.-% Borsäure und 20 Gew.-% Harnstoff in den Wirbelkühler eingedüst. Die Korngrösse des Gemisches liegt zwischen 0,8 bis 1,5 mm. Die Eindüsung erfolgte über 4 im gleichen Abstand rings um den Wirbelkühler angeordnete Düsen. Das Gemisch aus Harnstoff und Borsäure hat einen Schmelzbereich von 145 bis 176°C. Die pro Stunde zugeführte Menge beträgt 5 kg. Der Boroxidgehalt des Katalysators wird so zwischen 43% und 45% über 3 Wochen gehalten.

### Beispiel 3

Man verfährt wie in Beispiel 1 beschrieben. Anstelle von Borsäure wird ein Gemisch aus Bortrioxid und Caprolactam im Gewichtsverhältnis 80% Bortrioxid und 20% Caprolactam über 2 Düsen dem Katalysator zugeführt. Die pro Stunde an beiden Stellen zugeführte Menge betrug 3 kg. Der Bortrioxidgehalt des Katalysators hält sich bei etwa 41 bis 42%.

### Patentansprüche

1. Verfahren zur Herstellung von ε-Caprolactam durch katalytische Umlagerung von Cyclohexanonoxim in der Gasphase bei einer Temperatur von 230 bis 450°C an Bortrioxid enthaltenden Trägerkatalysatoren in der Wirbelschicht, wobei der Katalysator nach Massgabe seiner Erschöpfung der Wirbelschicht entnommen und mit molekularen Sauerstoff enthaltenden Gasen in aufgewirbeltem Zustand bei einer Temperatur von 600 bis 900°C behandelt und anschliessend vor Zurückführen in die Wirbelschicht dem Katalysator in aufgewirbeltem Zustand bei einer Temperatur von 300 bis 900°C Bortrioxid oder Borsäure zugesetzt wird, dadurch gekennzeichnet, dass man Bortrioxid oder Borsäure mit einer Korngrösse von 0,05 bis 1,5 mm an mindestens 2 Stellen dem aufgewirbelten Katalysator zuführt.

2. Verfahren nach Anspruch 1, dadurch gekennzeichnet, dass man dem Katalysator Bortrioxid oder Borsäure gemischt mit Caprolactam und/oder Harnstoff zuführt.

3. Verfahren nach den Ansprüchen 1 und 2, dadurch gekennzeichnet, dass man ein Gemisch aus 10 bis 99 Gew.-% Borsäure oder Bortrioxid berechnet als Bortrioxid sowie Caprolactam und/oder Harnstoff verwendet.

### Claims

1. A process for the preparation of ε-caprolactam by catalytic rearrangement of cyclohexanone-oxime in the gas phase at from 230 to 450°C over a supported catalyst, containing boron trioxide, in a fluidized bed, the catalyst being removed from the fluidized bed at the rate at which it becomes spent, and being treated, in a fluidized state, with gases containing molecular oxygen at from 600 to 900°C, after which boron trioxide or boric acid is added to the fluidized catalyst, at from 300 to 900°C, before the catalyst is returned to the fluidized bed, characterized in that boron trioxide or boric acid having a particle size of from 0.05 to 1.5 mm is fed to the fluidized catalyst at at least two points.

2. A process as claimed in claim 1, characterized in that boron trioxide or boric acid mixed with caprolactam and/or urea is fed to the catalyst.

3. A process as claimed in claims 1 and 2, characterized in that a mixture of from 10 to 99% by weight of boric acid (calculated as boron trioxide) or boron trioxide with caprolactam and/or urea is used.

### Revendications

1. Procédé de préparation d'ε-caprolactame par transposition catalytique de cyclohexano-oxime en phase gazeuse, à une température de 230 à 450°C, sur des catalyseurs à support contenant du trioxyde de bore, en couche en mouvement turbulent, le catalyseur étant prélevé de la couche turbulente en fonction de son épuisement, puis traité à l'étant de suspension turbulente et à une température de 600 à 900°C avec des gaz contenant de l'oxygène moléculaire, enfin le catalyseur, avant son retour dans la couche turbulente, étant additionné à une température de 300 à 900°C, de trioxyde de bore ou d'acide borique, caractérisé par le fait que l'on amène au catalyseur en mouvement turbulent, en au moins deux points, du trioxyde de bore ou de l'acide borique d'une grosseur de grains de 0,05 à 1,5 mm.

2. Procédé selon la revendication 1, caractérisé par le fait que l'on amène au catalyseur du trioxyde de bore ou de l'acide borique mélangé avec du caprolactame et/ou de l'urée.

3. Procédé selon les revendications 1 et 2, caractérisé par le fait que l'on utilise un mélange de 10 à 99% en poids d'acide borique ou de trioxyde de bore, calculé en trioxyde de bore ainsi que de caprolactame et/ou d'urée.